# FASCICULE DE BREVET EUROPEEN

(11) **EP 2 064 046 B1**
(45) Date de publication et mention de la délivrance du brevet: **02.12.2009**
(21) Numéro de dépôt: 07823445.7
(22) Date de dépôt: 23.08.2007
(51) Int. Cl.: B29C 45/16, A61L 9/12, A61L 9/01

(54) **PROCÉDÉ DE FABRICATION D 'UNE PIÈCE INJECTÉE DIFFUSANT DES ODEURS ET PIÈCE OBTENUE SELON CE PROCÉDÉ**
VERFAHREN ZUR HERSTELLUNG EINES EINSPRITZTEILS ZUR DUFTVERBREITUNG UND ANHAND DIESES VERFAHRENS GEWONNENES TEIL
METHOD OF MANUFACTURING AN INJECTED DIFFUSING PART FOR ODORS AND PART OBTAINED BY THIS METHOD

(30) Priorité: 29.08.2006 FR 0607606
(43) Date de publication de la demande: 03.06.2009
(73) Titulaire: Plastigray S.A.S., 70100 Gray (FR)
(72) Inventeur: SAVIN, Thierry, 70100 Arc Les Gray (FR); BOUVIER, Jean-Robert, 70100 Gray (FR)
(74) Mandataire: Nithardt, Roland
(86) Numéro de dépôt international: PCT/FR2007/001397
(87) Numéro de publication internationale: WO 2008/025896

(56) Documents cités:
- EP-A- 1 493 332
- EP-A1- 0 013 688
- WO-A-03/035368
- DE-B1- 1 802 684
- JP-A- 7 195 409
- US-A- 5 932 353
- US-A1- 2005 272 878
- US-B1- 6 394 094

## Description

### Domaine technique :

La présente invention concerne un procédé de fabrication d'une pièce injectée diffusant des odeurs, cette pièce étant réalisée en au moins deux matières thermoplastiques ayant des caractéristiques physiques différentes, procédé dans lequel on injecte simultanément ou séquentiellement dans un moule correspondant à ladite pièce au moins une première matière thermoplastique pour constituer une peau de la pièce et au moins une deuxième matière thermoplastique pour constituer un coeur de la pièce entouré de ladite peau.

Elle concerne également une pièce injectée diffusant des odeurs, cette pièce étant réalisée en au moins deux matières thermoplastiques ayant des caractéristiques physiques différentes par injection simultanée ou séquentielle dans un moule correspondant à ladite pièce d'au moins une première matière thermoplastique pour constituer une peau de la pièce et d'au moins une deuxième matière thermoplastique pour constituer un coeur de la pièce entouré de ladite peau.

### Technique antérieure :

Les techniques actuelles pour donner aux matières thermoplastiques la capacité de diffuser des fragrances sont complexes et souvent peu efficaces. Dans tous les cas, l'émission d'odeurs ne peut pas être contrôlée dans le temps. Dans une phase initiale, les odeurs émises sont trop prononcées puis, les odeurs émises sont diffusées de plus en plus faiblement pour s'estomper ou disparaître relativement rapidement.

Un exemple est donné dans le brevet européen EP 0 300 286, qui décrit un produit en matières synthétiques, sous la forme d'une feuille composite laminée comportant deux couches au moins, superposées et accolées par soudure ou collage, dont l'une est imperméable aux agents volatiles actifs d'une composition parfumante ou désodorisante et l'autre contient une composition parfumante ou désodorisante. Ce mode d'assemblage par collage ne permet pas de réaliser des pièces de formes complexes, économiques et en grande série. De plus, aucun dispositif n'est prévu pour contrôler la diffusion des odeurs.

Un autre exemple est donné dans le brevet européen EP 0 013 688, qui décrit un diffuseur de parfum constitué d'une pièce moulée dans une résine thermoplastique mélangée à un composé de cyclodextrine et de molécules odorantes. Etant donné que ce diffuseur ne comporte aucun moyen permettant de contrôler la diffusion des odeurs, sa durée de vie est limitée à environ un ou deux mois. Les mêmes inconvénients se retrouvent dans les dispositifs insecticides décrits dans les publications US 5 932 353, DE 1 802 684 et EP 1 493 332, qui ne comportent aucun moyen permettant de contrôler la diffusion de l'agent insecticide contenu dans une résine thermoplastique.

On connaît par ailleurs, notamment des publications JP 03 189140 et WO 03/035368, des récipients réalisés par injection multicouche de résines thermoplastiques, comportant une barrière aux gaz pour empêcher soit l'émission d'odeurs à travers les parois du récipient, soit la migration de l'oxygène en vue de préserver la qualité des produits contenus dans le récipient. Toutefois, cette technologie « barrière » largement utilisée dans l'emballage, qui a pour fonction de bloquer la migration des molécules gazeuses, n'est pas applicable à des pièces ayant pour fonction de diffuser des odeurs de manière contrôlée et maîtrisée.

D'autre part, la technique dite de l'injection sandwich est connue et consiste à injecter, au moyen de presses bi-injection, dans un moule simultanément ou séquentiellement deux polymères dont l'un forme la couche extérieure appelée peau et l'autre la partie centrale appelée coeur d'une pièce composite. Les polymères peuvent se présenter sous une forme solide ou une forme expansée ou sous une forme combinée. Les polymères peuvent être expansés par adjonction d'un agent porogène ou d'un gaz inerte ou par l'utilisation combinée de ces deux types de composants.

La présente invention se propose de pallier ces inconvénients en offrant un procédé de réalisation de pièces fabriquées par injection du type bi-matière, économique et permettant de donner à ces pièces plastiques des qualités efficaces et contrôlables d'émetteurs d'odeurs.

Ce but est atteint par le procédé tel que défini en préambule, caractérisé en ce que l'on utilise au moins pour ladite deuxième matière thermoplastique formant ledit coeur, un matériau qui comporte au moins une substance émettant des odeurs, et en ce que l'on utilise pour ladite première matière thermoplastique formant ladite peau, un matériau qui comporte des moyens filtrants agencés pour allonger le cheminement des molécules émises par ladite substance émettant des odeurs.

Ainsi, la matière de coeur constitue un réservoir de molécules odorantes et la matière de peau constitue un filtre qui permet de contrôler et de maîtriser la diffusion des odeurs pour qu'elle se prolonge dans le temps avec une excellente régularité de l'intensité odorante.

L'on peut utiliser pour ladite deuxième matière thermoplastique, formant ledit coeur, un matériau qui contient également des moyens filtrants pour allonger le cheminement de molécules émises par ladite substance émettant des odeurs.

Ces moyens filtrants peuvent être obtenus par greffage moléculaire de ladite matière thermoplastique ou par au moins un type d'éléments en occlusion dans ladite matière thermoplastique.

De manière avantageuse, l'on utilise pour lesdits éléments en occlusion des charges, telles que du talc micronisé, de la craie, du noir de carbone ou des nanoparticules, telles que l'argile montmorillonite.

Ce but est également atteint par une pièce injectée telle que définie en préambule, caractérisée en ce que ladite deuxième matière thermoplastique, formant-ledit coeur, comporte au moins une substance émettant des odeurs, et en ce que ladite première matière thermoplastique, formant ladite peau, comporte des moyens filtrants agencés pour allonger le cheminement de molécules émises par ladite substance émettant des odeurs.

Ladite deuxième matière thermoplastique, formant ledit coeur, peut également contenir des moyens filtrants pour allonger le cheminement de molécules émises par ladite substance émettant des odeurs.

La pièce obtenue constitue avantageusement une pièce d'habillage intérieur d'un véhicule parfaitement intégrée.

La présente invention et ses avantages seront mieux compris à la lecture de la description détaillée d'une forme de mise en oeuvre du procédé, et de la pièce obtenue selon l'invention, en référence aux dessins annexés donnés à titre indicatif et non limitatif, dans lesquels :

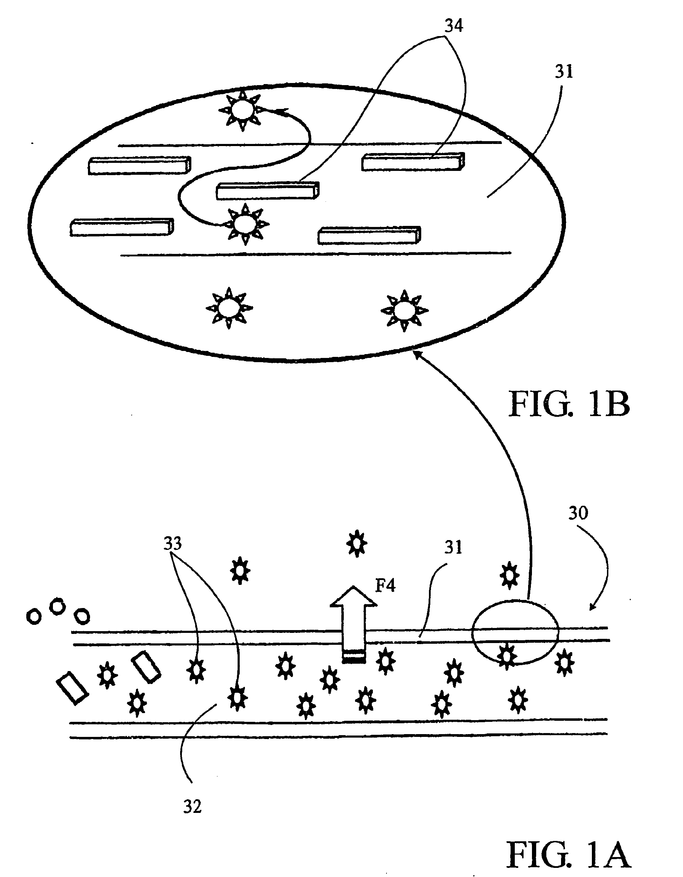

- la figure 1A est une vue schématique agrandie et partielle représentant une forme de réalisation d'une pièce selon l'invention et illustrant le mode de diffusion retardé d'une substance odorante, et
- la figure 1B est une vue schématique agrandie du détail B de la figure 1A.

En référence à la figure 1, la pièce 30 représentée partiellement et en coupe, comporte une peau 31 et un coeur 32 entouré de ladite peau 31. La peau 31 est réalisée par injection d'au moins une première matière thermoplastique et le coeur 32 est obtenu par l'injection d'au moins une deuxième matière thermoplastique. Selon la technique employée, la peau 31 et le coeur 32 peuvent être injectés séparément ou simultanément dans un moule qui correspond à la pièce 30 à fabriquer.

La peau 31 réalisée avec ladite première matière thermoplastique est perméable aux odeurs. Soit ce matériau présente directement cette caractéristique, soit il est traité avant ou pendant l'injection pour qu'il obtienne cette particularité qui lui permet de laisser passer des odeurs dans le sens des flèches F4.

Le coeur 32 réalisé avec la deuxième matière thermoplastique est également perméable aux odeurs. Soit ce matériau présente directement cette caractéristique, soit il est traité avant ou pendant l'injection pour qu'il obtienne cette particularité qui lui permet de laisser passer des odeurs dans le sens des flèches F4.

Le coeur 32 contient en plus des substances odorantes 33 en occlusion dans la masse et constitue un réservoir d'odeurs. Ces substances odorantes 33 connues sont des parfums ou des molécules, qui peuvent posséder un noyau aromatique, et qui sont mélangées à ladite deuxième matière thermoplastique avant ou pendant la phase d'injection. Les substances odorantes 33 peuvent être des mélanges de parfum, des produits d'origine naturelle ou synthétique, des mélanges de produits qui diffusent des odeurs en permanence ou lorsqu'elles sont sollicitées par une contrainte extérieure telle qu'une pression par exemple. La proportion et le mélange de substances odorantes 33 dépendent des effets recherchés. La consistance, la densité et la perméabilité de la deuxième matière thermoplastique sont choisies en fonction de la vitesse de diffusion des odeurs que l'on souhaite obtenir.

Le but recherché par l'invention est de conserver cette caractéristique de diffusion des odeurs pendant une durée aussi longue que possible et que l'intensité de la diffusion soit régulière pendant toute la durée de vie du produit.

Afin de ralentir la diffusion des molécules odorantes 33 et par conséquent de prolonger le caractère du produit 30 à « parfumer » l'air environnant, la peau. 31 est réalisée dans une première matière thermoplastique comportant des moyens filtrants capables d'augmenter la longueur du trajet de cheminement des molécules odorantes 33 à travers la peau 31. Bien entendu, ces moyens filtrants peuvent également être prévus dans la deuxième matière thermoplastique formant le coeur 32, en complément de ceux prévus dans la peau 31, pour augmenter encore l'effet ralentisseur.

Ces moyens filtrants peuvent être obtenus par des éléments 34 en occlusion dans ladite matière. Ces éléments 34 en occlusion peuvent être des charges minérales, telles que du talc micronisé, de la craie, du noir de carbone, ou similaire, mélangées à la matière de peau à hauteur de 20 à 40% en poids par exemple. Ces éléments 34 en occlusion peuvent être aussi des nanoparticules, telles que de l'argile montmorillonite ou similaire, mélangées à la matière de peau à hauteur de quelques pourcents. Ces moyens filtrants peuvent être aussi obtenus par greffage moléculaire de ladite matière thermoplastique. De ce fait, la diffusion des molécules odorantes 33 est nettement plus lente et plus durable. De même, l'intensité olfactive est régulière dans le temps, étant donné que les moyens filtrants permettent de réguler cette diffusion.

Une des applications spécifique concerne l'industrie automobile, pour laquelle on cherche à supprimer l'odeur naturelle des pièces plastiques utilisées pour l'habillage intérieur du véhicule, qui n'est pas agréable. Une des solutions consiste à compenser cette mauvaise odeur en ajoutant une odeur agréable. Il est connu d'ajouter dans l'habitacle du véhicule un diffuseur d'odeurs spécifique, accroché au rétroviseur, clippé dans un aérateur, ou collé sur le tableau de bord. Toutefois, ces diffuseurs d'odeurs ont une durée de vie très limitée, environ un mois, et l'intensité de l'odeur décroit rapidement. Le procédé de l'invention permet d'ajouter très facilement et à moindre coût une odeur agréable dans une des pièces d'habillage du véhicule, comme par exemple, dans les tapis de console ou d'agrément, qui sont des pièces discrètes, parfaitement intégrées dans l'habitacle et facilement interchangeables. Avec ce procédé, on obtient une diffusion d'odeurs très agréable, d'intensité olfactive régulière, sur une très longue période, pouvant atteindre plus de deux ans.

La présente invention n'est pas limitée aux formes de réalisations décrites, mais peut s'étendre à toutes sortes de réalisation du type pièces injectées en bi-matière ou multi-matière thermoplastique, notamment thermoplastique élastomère, d'une manière évidente pour l'homme du métier, sans sortir du champ de la présente invention. Par exemple et en fonction du type de pièce à réaliser, la peau 31 peut être composée d'une première couche de matière comportant lesdits moyens filtrants, recouverte d'une seconde couche de matière formant une peau d'aspect. Les formes et les caractéristiques physiques peuvent être variées indéfiniment en fonction des besoins et des applications. Les odeurs diffusées peuvent être très diverses en fonction des effets que l'on souhaite obtenir. Le champ d'application de la présente invention est très vaste et intéresse notamment les industries de l'automobile, de l'aviation, du bâtiment, de l'ameublement, etc.

## Revendications

1. Procédé de fabrication d'une pièce (30) injectée diffusant des odeurs, cette pièce étant réalisée en au moins deux matières thermoplastiques ayant des caractéristiques physiques différentes, procédé dans lequel l'on injecte simultanément ou séquentiellement dans un moule correspondant à ladite pièce au moins une première matière thermoplastique pour constituer une peau extérieure (31) de la pièce et au moins une deuxième matière thermoplastique pour constituer un coeur (32) de la pièce entouré de ladite peau, et dans lequel l'on utilise au moins pour ladite deuxième matière thermoplastique formant ledit coeur, un matériau qui comporte au moins une substance (33) émettant des odeurs, **caractérisé en ce que** l'on utilise pour ladite première matière thermoplastique formant ladite peau, un matériau qui comporte des moyens filtrants agencés pour allonger le cheminement de molécules émises par ladite substance émettant des odeurs.

2. Procédé selon la revendication 1, **caractérisé en ce que** l'on utilise pour ladite deuxième matière thermoplastique formant ledit coeur, un matériau qui comporte également des moyens filtrants agencés pour allonger le cheminement de molécules émises par ladite substance émettant des odeurs.

3. Procédé selon l'une quelconque des revendications précédentes, **caractérisé en ce que** l'on utilise pour lesdits moyens filtrants un greffage moléculaire de ladite matière thermoplastique.

4. Procédé selon l'une quelconque des revendications 1 à 2, **caractérisé en ce que** l'on utilise pour lesdits moyens filtrants au moins un type d'éléments (34) en occlusion dans ladite matière thermoplastique.

5. Procédé selon la revendication 4, **caractérisé en ce que** l'on utilise pour lesdits éléments en occlusion des charges ou des nanoparticules.

6. Procédé selon la revendication 5, **caractérisé en ce que** lesdits éléments en occlusion sont choisis parmi le talc micronisé, la craie, le noir de carbone pour les charges, et parmi l'argile montmorillonite pour les nanoparticules.

7. Pièce injectée (30) diffusant des odeurs, cette pièce étant réalisée en au moins deux matières thermoplastiques ayant des caractéristiques physiques différentes, obtenue par injection simultanée ou séquentielle dans un moule correspondant à ladite pièce d'au moins une première matière thermoplastique pour constituer une peau extérieure (31) de la pièce et d'au moins une deuxième matière thermoplastique pour constituer un coeur (32) de la pièce entouré de ladite peau, ladite deuxième matière thermoplastique au moins, formant ledit coeur, comportant au moins une substance (33) émettant des odeurs, **caractérisée en ce que** ladite première matière thermoplastique, formant ladite peau, comporte des moyens filtrants agencés pour allonger le cheminement de molécules émises par ladite substance émettant des odeurs.

8. Pièce injectée selon la revendication 7, **caractérisée en ce que** ladite deuxième matière thermoplastique, formant ledit coeur, comporte également des moyens filtrants pour allonger le cheminement de molécules émises par ladite substance émettant des odeurs.

9. Pièce injectée selon l'une quelconque des revendications 7 à 8, **caractérisée en ce que** lesdits moyens filtrants sont obtenus par greffage moléculaire de ladite matière thermoplastique.

10. Pièce injectée selon l'une quelconque des revendications 7 à 8, **caractérisée en ce que** lesdits moyens filtrants comportent au moins un type d'éléments (34) en occlusion dans ladite matière thermoplastique.

11. Pièce injectée selon la revendication 10, **caractérisée en ce que** lesdits éléments en occlusion comportent des charges ou des nanoparticules.

12. Pièce selon la revendication 11, **caractérisée en ce que** lesdits éléments en occlusion sont choisis parmi le talc micronisé, la craie, le noir de carbone pour les charges, et parmi l'argile montmorillonite pour les nanoparticules.

13. Pièce selon l'une quelconque des revendications 7 à 12, **caractérisée en ce qu'**elle constitue une pièce d'habillage intérieur d'un véhicule.

## Claims

1. Method of manufacturing an injected part (30) diffusing odours, this part being manufactured out of at least two thermoplastic materials having different physical characteristics, method in which one injects simultaneously or sequentially in a form that corresponds to said part at least one first thermoplastic material to form an external skin (31) of the part and at least one second thermoplastic material to form a core (32) of the part surrounded by said skin, and in which one uses at least for said second thermoplastic material forming said core a material that includes at least one substance (33) giving off odours, **characterized in that** one uses, for said first thermoplastic material forming said core, a material that includes filtering means arranged to lengthen the path of the molecules given off by said substance giving off odours.

2. Method according to claim 1, **characterized in that** one uses, for said second thermoplastic material forming said core, a material that also includes filtering means arranged to lengthen the path of the molecules given off by said substance giving off odours.

3. Method according to any of the previous claims, **characterized in that** one uses, for said filtering means, a molecular grafting of said thermoplastic material.

4. Method according to any of the claims1 to 2, **characterized in that** one uses, for said filtering means, at least one type of elements (34) in occlusion in said thermoplastic material.

5. Method according to claim 4, **characterized in that** one uses, for said elements in occlusion, fillers or nano-particles.

6. Method according to claim 5, **characterized in that** said elements in occlusion are chosen among the micronized talc, the chalk, the carbon black for the fillers, and among the montmorillonite clay for the nano-particles.

7. Injected part (30) diffusing odours, this part being manufactured out of at least two thermoplastic materials having different physical characteristics, obtained by simultaneous or sequential injection in a form that corresponds to said part of at least one first thermoplastic material to form an external skin (31) of the part and of at least one second thermoplastic material to form a core (32) of the part surrounded by said skin, said second thermoplastic material, at least one, forming said core, including at least one substance (33) giving off odours, **characterized in that** said first thermoplastic material, forming said core, includes filtering means arranged to lengthen the path of the molecules given off by said substance giving off odours.

8. Injected part according to claim 7, **characterized in that** said second thermoplastic material , forming said core, also includes filtering means to lengthen the path of the molecules given off by said substance giving off odours.

9. Injected part according to any of claims 7 to 8, **characterized in that** said filtering means are obtained by molecular grafting of said thermoplastic material.

10. Injected part according to any of claims 7 to 8, **characterized in that** said filtering means include at least one type of elements (34) in occlusion in said thermoplastic material.

11. Injected part according to claim 10, **characterized in that** said elements in occlusion include fillers or nano-particles.

12. Part according to claim 11, **characterized in that** said elements in occlusion are chosen among the micronized talc, the chalk, the carbon black for the fillers, and among the montmorillonite clay for the nano-particles.

13. Part according to any of the claims 7 to 12, **characterized in that** it forms an inside lining part of a vehicle.

## Patentansprüche

1. Verfahren zur Herstellung eines Einspritzteils (30) zur Duftverbreitung, wobei dieses Teil aus mindestens zwei thermoplastischen Stoffen hergestellt wird, die unterschiedliche physische Eigenschaften aufweisen, Verfahren in dem man gleichzeitig oder sequentiell in eine dem besagten Teil entsprechende Form mindestens einen ersten thermoplastischen Stoff einspritzt, um eine Außenhaut (31) des Teils zu bilden, und mindestens einen zweiten thermoplastischen Stoff einspritzt, um einen in der besagten Haut eingeschlossenen Kern (32) des Teils zu bilden, und in dem man mindestens für den den besagten Kern bildenden besagten zweiten thermoplastischen Stoff ein Material verwendet, das mindestens eine Duftverbreitende Substanz (33) beträgt, **dadurch gekennzeichnet, dass** man für den besagten ersten thermoplastischen Stoff, der die besagte Haut bildet, ein Material verwendet, das filternde Mittel beträgt, die angeordnet sind, um den Weg der von der besagten Duftverbreitenden Substanz abgegebenen Moleküle zu verlängern.

2. Verfahren nach Anspruch 1, **dadurch gekennzeichnet, dass** man für den besagten zweiten thermoplastischen Stoff, der den besagten Kern bildet, ein Material verwendet, das ebenfalls filternde Mittel beträgt, die angeordnet sind, um den Weg der von der besagten Duftverbreitenden Substanz abgegebenen Moleküle zu verlängern.

3. Verfahren nach einem der vorhergehenden Ansprüche, **dadurch gekennzeichnet, dass** man für die besagten filternden Mittel eine molekulare Pfropfung des besagten thermoplastischen Stoffs verwendet.

4. Verfahren nach einem der Ansprüche1 bis 2, **dadurch gekennzeichnet, dass** man für die besagten filternden Mittel mindestens einen im besagten thermoplastischen Stoff eingeschlossenen Typ Elemente (34) verwendet.

5. Verfahren nach Anspruch 4, **dadurch gekennzeichnet, dass** man für die besagten eingeschlossenen Elemente Füllstoffe oder Nanopartikel verwendet.

6. Verfahren nach Anspruch 5, **dadurch gekennzeichnet, dass** die besagten eingeschlossenen Elemente für die Füllstoffe unter dem mikronisiertem Talkum, der Kreide, dem Russ, und für die Nanopartikel unter dem Montmorillonit-Ton gewählt werden.

7. Duftverbreitendes Einspritzteil (30), wobei dieses Teil aus mindestens zwei thermoplastischen Stoffen hergestellt wird, die unterschiedliche physische Eigenschaften aufweisen, hergestellt durch gleichzeitiges oder sequentielles Einspritzen in eine dem besagten Teil entsprechende Form von mindestens einem ersten thermoplastischen Stoff, um eine Außenhaut (31) des Teils zu bilden, und von mindestens einem zweiten thermoplastischen Stoff, um einen in der besagten Haut eingeschlossenen Kern (32) des Teils zu bilden, wobei der besagte zweite thermoplastische Stoff, mindestens einer, der den besagten Kern bildet, mindestens eine Duftverbreitende Substanz (33) beträgt, **dadurch gekennzeichnet, dass** der besagte erste thermoplastische Stoff, der die besagte Haut bildet, filternde Mittel beträgt, die angeordnet sind, um den Weg der von der besagten Duftverbreitenden Substanz abgegebenen Moleküle zu verlängern.

8. Einspritzteil nach Anspruch 7, **dadurch gekennzeichnet, dass** der besagte zweite thermoplastische Stoff, der den besagten Kern bildet, ebenfalls filternde Mittel beträgt um den Weg der von der besagten Duftverbreitenden Substanz abgegebenen Moleküle zu verlängern.

9. Einspritzteil nach einem der Ansprüche 7 bis 8, **dadurch gekennzeichnet, dass** die besagten filternden Mittel durch molekulare Pfropfung des besagten thermoplastischen Stoffs erhalten werden.

10. Einspritzteil nach einem der Ansprüche 7 bis 8, **dadurch gekennzeichnet, dass** die besagten filternden Mittel mindestens einen im besagten thermoplastischen Stoff eingeschlossenen Typ Elemente (34) betragen.

11. Einspritzteil nach Anspruch 10, **dadurch gekennzeichnet, dass** die besagten eingeschlossenen Elemente Füllstoffe oder Nanopartikel betragen.

12. Teil nach Anspruch 11, **dadurch gekennzeichnet, dass** die besagten eingeschlossenen Elemente für die Füllstoffe unter dem mikronisiertem Talkum, der Kreide, dem Russ, und für die Nanopartikel unter dem Montmorillonit-Ton gewählt werden.

13. Teil nach einem der Ansprüche 7 bis 12, **dadurch gekennzeichnet, dass** es ein Innenverkleidungs-Teil eines Fahrzeugs bildet.
